# EUROPEAN PATENT SPECIFICATION

(11) **EP 2 985 586 B1**
(45) Date of publication and mention of the grant of the patent: **19.12.2018**
(21) Application number: 14782403.1
(22) Date of filing: 27.02.2014
(51) Int. Cl.: G01N 1/04, B01J 19/00, C12M 1/00, G01N 1/28, G01N 27/447, B01L 3/00, B26D 5/02, B65B 35/00, B65B 3/04

(54) **GEL PLATE SEGMENTING AND DISPENSING DEVICE AND SEGMENTING AND DISPENSING METHOD**
GELPLATTENSEGMENTIERUNGS- UND -AUSGABEVORRICHTUNG UND SEGMENTIERUNGS- UND AUSGABEVERFAHREN
DISPOSITIF DE SEGMENTATION ET DE DISTRIBUTION DE PLAQUE DE GEL ET PROCÉDÉ DE SEGMENTATION ET DE DISTRIBUTION

(30) Priority: 11.04.2013 JP 2013082651
(43) Date of publication of application: 17.02.2016
(73) Proprietor: Syoryukensetsu Corporation, Nagano-shi, Nagano3800916 (JP)
(72) Inventor: NAGANO, Tetsuo, Tokyo 167-0032 (JP); KOMATSU, Toru, Kodaira-shi Tokyo 187-0045 (JP); KONNO, Satoru, Kasukabe-shi Saitama 334-0064 (JP); SIMODA, Masahiro, Tokyo 120-0034 (JP)
(74) Representative: Lang, Johannes
(86) International application number: PCT/JP2014/054953
(87) International publication number: WO 2014/167911

(56) References cited:
- EP-A1- 1 384 993
- EP-A1- 1 944 603
- WO-A1-2005/029061
- WO-A1-2010/088517
- JP-A- H07 506 421
- JP-A- S63 119 668
- JP-A- 2002 525 191
- JP-A- 2007 192 732
- JP-A- 2007 524 069
- JP-A- 2009 509 127
- US-A1- 2007 119 712

## Description

### Technical Field

The present invention relates to a fragmentation and dispensing apparatus of a gel plate and a fragmentation and dispensing method of the gel plate. More specifically, the invention relates to an apparatus and a method for cutting a gel plate into gel fragments after electrophoresis used for analysis of protein or DNA (deoxyribonucleic acid) and dispensing the gel fragments into respective wells of a microtiter plate.

### Background Art

Recently, two-dimensional electrophoresis is often applied for protein analysis. Most typically, in first dimension electrophoresis, proteins are separated by isoelectric point electrophoresis using a polyacrylamide strip gel. Then, in second dimension electrophoresis, the proteins are separated based on molecular weight using electrophoresis (SDS-PAGE, negative electrophoresis, or the like) employing a polyacrylamide slab gel.

Then, by applying gel staining such as CBB (Coomassie Brilliant Blue) staining or fluorescent staining to the polyacrylamide gel after the two-dimensional electrophoresis, it is so arranged that the proteins in the gel are visually detected. Then, by using a method of cutting down a portion of the stained gel by a knife or a method of pressing a hollow metal pipe against the gel to hollow out the gel, each protein spot is extracted as a sample.

The protein in the sample is thereafter identified by a method referred to as in-gel digestion. That is, the protein is fragmented using a protease such as trypsin. Peptides produced from fragmentation of the protein are collected to measure the molecular weight of the peptides using a mass spectrometer. Then, based on a result of the measurement, the protein is identified by a search using a database of amino acid sequences, which is referred to as a peptide mass finger printing (PMF) method.

The above description was directed to the protein analysis. Agarose gel is mainly employed for DNA electrophoresis.

A lot of proposals have been hitherto made on a method of extracting a sample from a gel plate after the electrophoresis using the polyacrylamide gel or the agarose gel.

Patent Document 1 to be listed below, for example, discloses a "gel cut-out device". In the gel cut-out device", a cylindrical gel cut-out section is attached to the tip portion of a pipetting device. A stained portion of a gel plate is cut out by a tip cutting edge of the gel cut-out section, and the gel portion cut out by depressurizing the inside of the gel cut-out section is held in front of a gel stopper. Moreover, by pressurizing the inside of the gel cut-out section to the contrary, the gel portion may be extruded from the gel cut-out section to an outside.

Patent Document 2 to be listed below discloses a " method of isolating a sample from a gel" constituted from a step of pressing a tip opening portion of a hollow disposable chip against a stained portion of a gel plate to hollow out the stained portion, a step of putting the hollowed-out gel fragment into a reaction vessel together with the chip with the hollowed-out chip held by the chip, and a step of collecting a target biopolymer from the gel fragment in the reaction vessel using a chemical treatment liquid.

Further, Patent Documents 3 and 4 to be listed below respectively disclose a "sample isolation device using gel electrophoresis" and a "gel, a method, and a device for identifying and characterizing biomolecules". The device disclosed in each of Patent Documents 3 and 4 includes means for optically recognizing an electrophoresis pattern developed in a gel plate and a working arm mechanism operable to move a cut-out tool to X, Y, and Z directions to cut out a predetermined portion of the gel plate. The device disclosed in each of Patent Documents 3 and 4 can automatically isolate a sample from a desired region of the gel plate and can move the sample to a microtiter plate or the like.

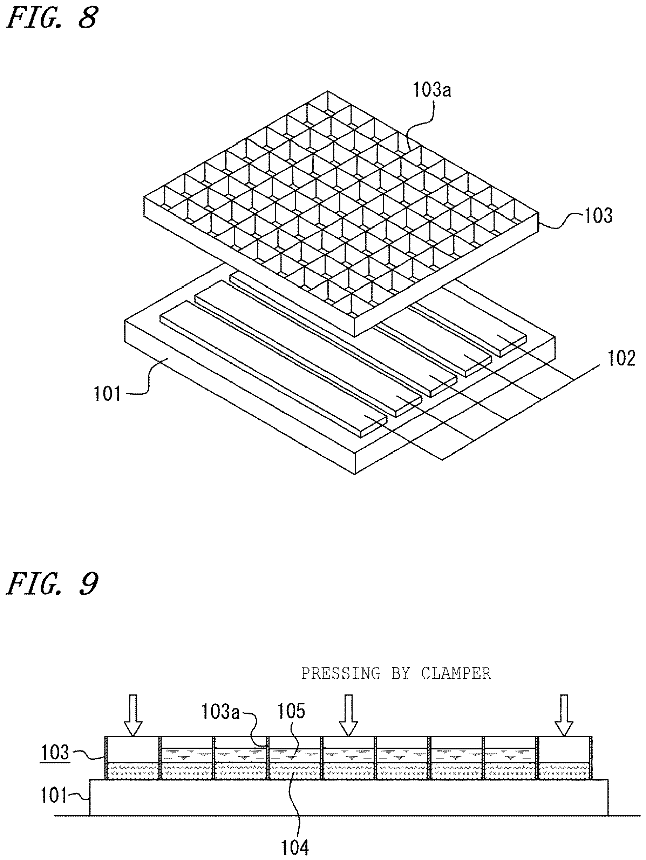

Meanwhile, different from the method of selectively cutting out only a portion of the gel plate and then transferring the cut-out portion to an analysis process as disclosed in each of the above-mentioned Patent Documents 1 to 4, Patent Document 5 to be listed below also proposes a "method and a device for extracting molecules using a frame" as shown in Figs. 8 and 9.

Specifically, by pressing a bottomless microtiter plate (frame) 103 having a lattice-shaped thin wall 103a formed thereon against a gel plate 102 placed on the surface of a flat plate 101 made of plastic or the like, the gel plate 102 is divided into gel fragments 104 of respective small segments. Then, a solvent 105 is individually applied to a gel fragment 104 in each segment, thereby extracting molecules included in each gel fragment 104.

### Citation List

### Patent Documents

Patent Document 1: Japanese Unexamined Patent Application Publication No. H7-132079
Patent Document 2: Japanese Patent No. 4378821
Patent Document 3: Japanese Unexamined Patent Application Publication No. H9-15206
Patent Document 4: Japanese Translation of PCT International Application Publication No. JP-T-2001-504938 (WO98/23950)
Patent Document 5: Japanese Translation of PCT International Application Publication No. JP-T-2007-524069 (WO2005/029061)

### Summary of Invention

### Technical Problem

According to the method proposed in the above-mentioned Patent Document 5, the entirety of the gel plate 102 after electrophoresis may be divided into the gel fragments 104 in the form of a matrix, and each small segment surrounded by the thin wall 103a of the microtiter plate (frame) 103 can be used as a Petri dish without alteration. The molecules included in each gel fragment 104 may be thereby extracted and analyzed.

Further, the process can be performed in a state where a planar positional relationship of a region stained by a color reaction reagent relative to the gel plate 102 after the electrophoresis is maintained without alteration.

Thus, according to the method proposed in the above-mentioned Patent Document 5, a lot of data may be obtained in an environment where mutual comparison of the lot of data under a same condition is easy, and an analysis work may be made more efficient, as compared with the technology proposed in each of the above-mentioned Patent Documents 1 to 4 where a specific region of the gel plate is individually cut out to be moved to a place for analysis of the specific region. The method proposed in Patent Document 5 includes a functional advantage of a common microtiter plate.

In the method proposed in the above-mentioned Patent Document 5, however, when the microtiter plate (frame) 103 is pressed against the surface of the flat plate 101 while cutting the gel plate 102, there is instability of work such as lateral sliding of the microtiter plate (frame) 103 due to interposition of the gel plate 102.

Entire end surfaces of the thin wall 103a of the microtiter plate (frame) 103 need to be adhered to the surface of the flat board 101 in order to completely separate the respective small segments from one another.

Accordingly, a clamping operation by a clamper (not shown) for strongly pressing the microtiter plate (frame) 103 against the flat plate 101 becomes necessary, and the clamper itself also obstructs the analysis work.

Further, though not mentioned in Patent Document 5, there is a disadvantage that flatness and smoothness with a high precision are demanded for the end surfaces of the thin wall 103a and the surface of the flat plate 101 in order to achieve the above-mentioned purpose.

Then, the present invention has been made with an object of providing a fragmentation and dispensing apparatus of a gel plate and a method of fragmenting and dispensing the gel plate operable to rationally solve the above-mentioned problems by utilizing simplicity and a functional advantage of a microtiter plate.

### Solution to Problem

An apparatus according to the invention of the present application relates to a fragmentation and dispensing apparatus of a gel plate according to claim 1. The fragmentation and dispensing apparatus may include:
a microtiter plate including a lattice-shaped partition wall with concave portions thereof surrounded by the lattice-shaped partition wall and arranged in the form of a matrix, end portions of the lattice-shaped partition wall being formed to be sharp; and
a pressing tool including fitting means with respect to the microtiter plate and including a lattice-shaped wall portion or a lattice-shaped convex portion formed thereon, the lattice-shaped wall portion or the lattice-shaped convex portion having a flat surface or a curved surface configured to be opposed to and abut against each end portion of the lattice-shaped partition wall of the microtiter plate when the pressing tool is fitted onto the microtiter plate; and
by fitting the pressing tool onto the microtiter plate and pressing a gel plate against the microtiter plate with the gel plate placed on the lattice-shaped partition wall of the microtiter plate, the gel plate is divided into fragments and the fragments are then dispensed into the respective concave portions of the microtiter plate.

A method according to the invention of the present application relates to a fragmentation and dispensing method of a gel plate using a microtiter plate and a pressing tool according to claim 5. The microtiter plate includes a lattice-shaped partition wall with concave portions thereof surrounded by the lattice-shaped partition wall and arranged in the form of a matrix. End portions of the lattice-shaped partition wall are formed to be sharp. The pressing tool includes fitting means with respect to the microtiter plate and includes a lattice-shaped wall portion or a lattice-shaped convex portion formed thereon. The lattice-shaped wall portion or the lattice-shaped convex portion has a flat surface or a curved surface configured to be opposed to and abut against each end portion of the lattice-shaped partition wall of the microtiter plate when the pressing tool is fitted onto the microtiter plate. The fragmentation and dispensing method may include:
a first step of placing a gel plate on the end portions of the lattice-shaped partition wall of the microtiter plate; and
a second step of fitting the pressing tool onto the microtiter plate to press the gel plate against the microtiter plate, thereby dividing the gel plate placed in the first step into fragments and then dispensing the fragments into the respective concave portions of the microtiter plate.

According to each invention of the present application, when the pressing tool is fit onto the microtiter plate, the gel plate is sandwiched and cut between the sharp end portions of the partition wall on the side of the microtiter plate and the surfaces (flat surfaces or curved surfaces) of the wall portion or the convex portion on the side of the pressing tool that have abutted against the sharp end portions. The fragments of the gel plate resulting from the division are respectively pressed into the concave portions of the microtiter plate and are then dispensed, while maintaining the positional relationship of the fragments in the gel plate before the division without alteration.

In this case, each end portion of the microtiter plate serves as a cutting edge. Thus, it may be so arranged that the pressing tool is formed of just a flat plate and the pressing tool wholly presses the gel plate. The gel plate, however, has a comparatively strong adherence. Accordingly, when the pressing tool is formed of the flat plate and when the pressing tool is removed from the microtiter plate after division of the gel plate, each fragment resulting from the division is attached to the flat surface of the pressing tool. Thus, the fragment cannot be dispensed into each concave portion of the microtiter plate.

For that reason, the pressing tool is also formed with the lattice-shaped wall portion or the lattice-shaped convex portion associated with the lattice-shaped partition wall of the microtiter plate. A disadvantage to be caused by the adherence of the gel plate is thereby solved.

The fragmentation and dispensing function for the gel plate according to the present invention is associated with a state of the abutment surface of the lattice-shaped wall portion or the lattice-shaped convex portion of the pressing tool configured to abut against each end portion of the lattice-shaped partition wall of the microtiter plate. In order to appropriately press the gel plate, the abutment surface needs to have a certain width or more. A contact area between the abutment surface and the gel plate in the process of sandwiching and cutting the gel plate cannot be set to be so large in order to reduce influence of the adhesive force of the gel plate and implement stable dispensing.

Preferably, either one of the following sets of configuration and condition is set for the pressing tool, in terms of this respect:
(a) the abutment part of the lattice-shaped wall portion or the lattice-shaped convex portion of the pressing tool configured to abut against each end portion of the lattice-shaped partition wall of the microtiter plate is formed as a flat end surface having a predetermined width, and the width of the end surface is larger than the width of a section of the end portion of the lattice-shaped partition wall of the microtiter plate corresponding to a cutting edge, and the width of the end surface is set within a range that does not cause adhesion of a gel to the pressing tool due to adherence of the gel constituting the gel plate and divided into the fragments when the pressing tool is released from the state of being fitted on the microtiter plate.
(b) the abutment part of the lattice-shaped wall portion or the lattice-shaped convex portion of the pressing tool configured to abut against each end portion of the lattice-shaped partition wall of the microtiter plate is formed as a convex articulate surface, and the curvature of the articulate surface is set within a range that does not cause adhesion of a gel to the pressing tool due to adherence of the gel constituting the gel plate and divided into the fragments when the pressing tool is released from the state of being fitted on the microtiter plate.

It is rational and is also simple for actual use to set the fitting means of the pressing tool with respect to the microtiter plate to a mechanism whereby an outer peripheral frame portion of the pressing tool is fit onto an outer peripheral side surface of the microtiter plate.

The gel that has been fragmented after the division is pressed into the respective concave portions of the microtiter plate. The pressed gel often remains in the vicinity of opening sections of the respective concave portions due to adherence of the gel. Thus, when a solvent is injected for analysis of the gel inside each concave portion, the injection may be obstructed.

In that case, the gel that has been fragmented may also be pressed into the respective concave portions with a pipette or a slim stick. However, it may be so arranged that a centrifuge or the like is used to cause a centrifugal force to act on the fragments of the gel plate dispensed into the respective concave portions of the microtiter plate in a direction toward bottom portions of the respective concave portions, thereby depositing the fragments of the gel plate on bottom sides of the respective concave portions.

### Advantageous Effects of Invention

"The fragmentation and dispensing apparatus of a gel plate and the fragmentation and dispensing method of the gel plate" of the present invention make it possible to cut the gel plate into fragments and then dispense the fragments into the concave portions (wells) of the microtiter plate by using the microtiter plate and the pressing tool and by an extremely simple operation.

According to the present invention, a transition to the microtiter plate is made while maintaining the two-dimensional positional relationship of each fragmented gel in the gel plate without alteration. Thus, a lot of data are readily compared with one another under a same condition. Further, each concave portion includes a bottom. Accordingly, different from the case of the above-mentioned Patent Document 5, independence between the concave portions that are adjacent to each other may be clearly maintained without using a special tool such as a clamper. Improvement in reliability of analysis work and improved efficiency of the analysis work are achieved.

### Brief Description of Drawings

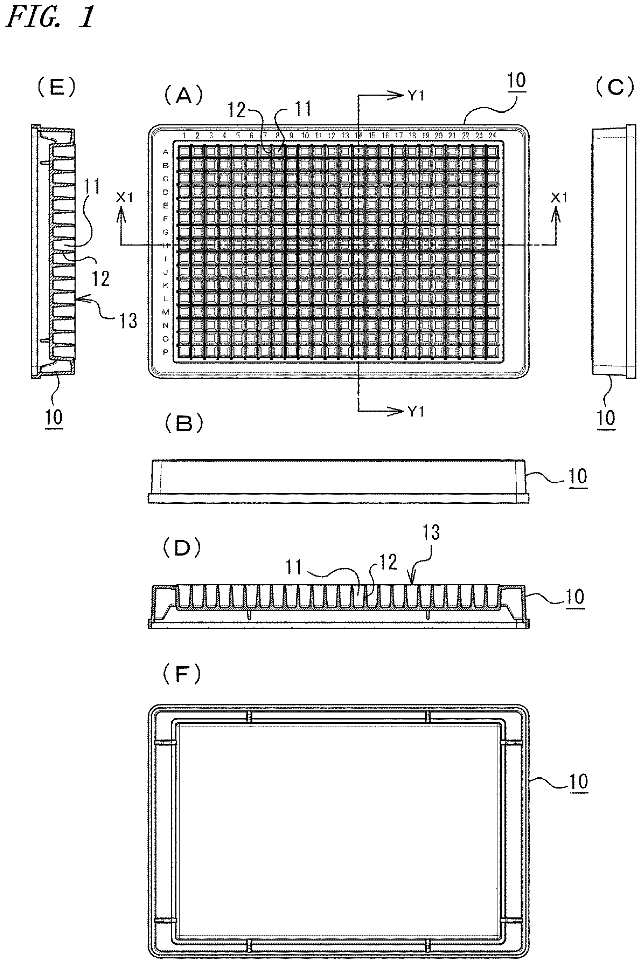

Figs. 1A to 1F show a microtiter plate according to the present invention, wherein Fig. 1A is a plan view, Fig. 1B is a front view, Fig. 1C is a right side view, Fig. 1D is a sectional view taken along arrow X1 - X1 in Fig. 1A, Fig. 1E is a sectional view taken along arrow Y1 - Y1 in Fig. 1A, and Fig. 1F is a bottom view.

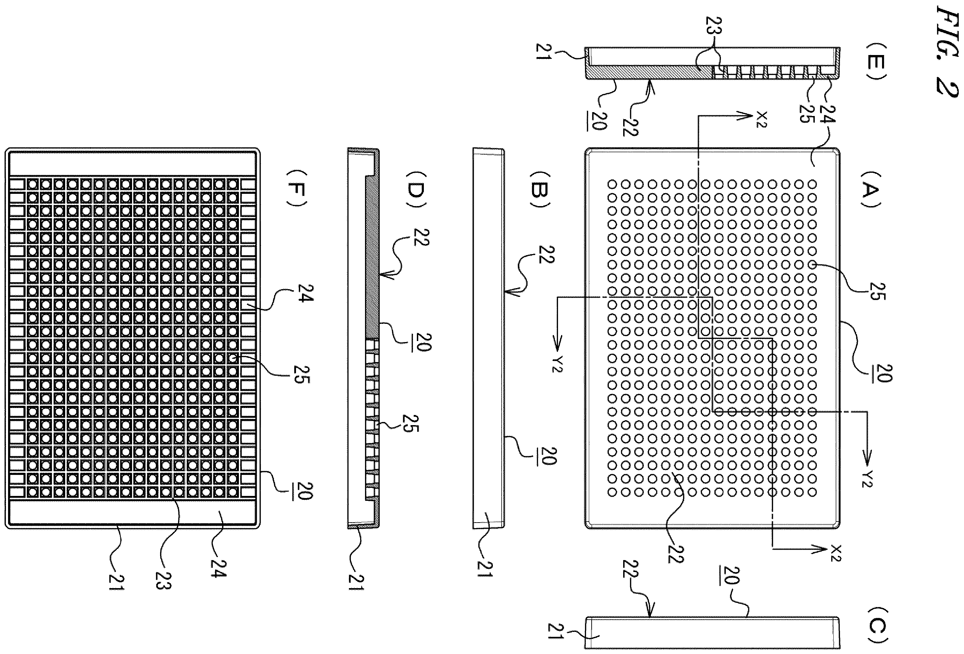

Figs. 2A to 2F show a pressing tool according to the present invention, wherein Fig. 2A is a plan view, Fig. 2B is a front view, Fig. 2C is a right side view, Fig. 2D is a sectional view taken along arrow X2 - X2 in Fig. 2A, Fig. 2E is a sectional view taken along arrow Y2 - Y2 in Fig. 2A, and Fig. 2F is a bottom view.

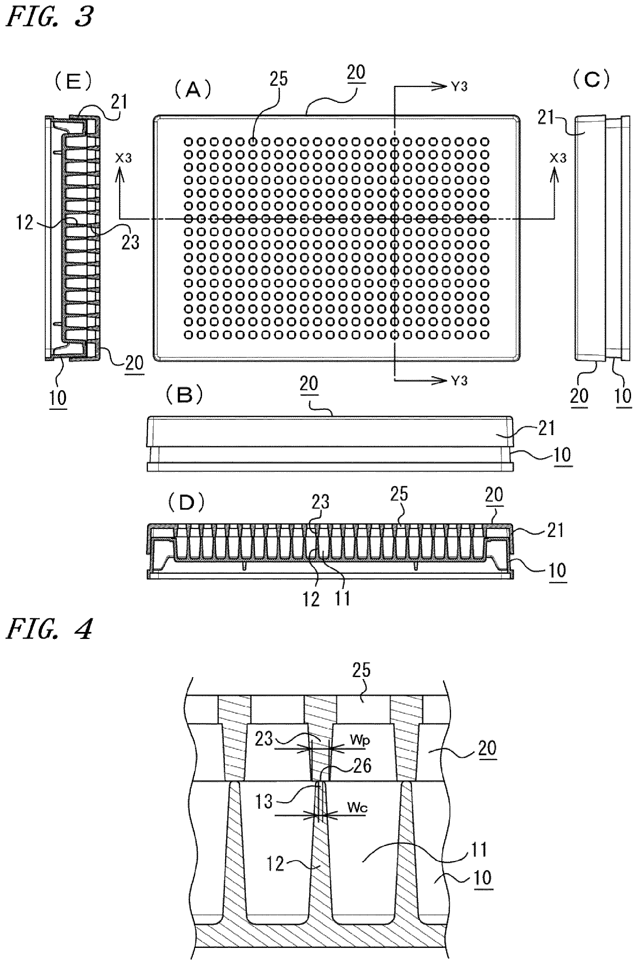

Figs. 3A to 3E show a state where the pressing tool is fit on the microtiter plate, wherein Fig. 3A is a plan view, Fig. 3B is a front view, Fig. 3C is a right side view, Fig. 3D is a sectional view taken along arrow Y3 - Y3 in Fig. 3A, and Fig. 3E is a sectional view taken along arrow X3 - X3 in Fig. 3A.
Fig. 4 is a sectional view enlarging a pertinent portion showing an abutting state between each end portion of a lattice-shaped partition wall of the microtiter plate and a corresponding end surface of a lattice-shaped partition wall of the pressing tool in the state where the pressing tool is fit on the microtiter plate.

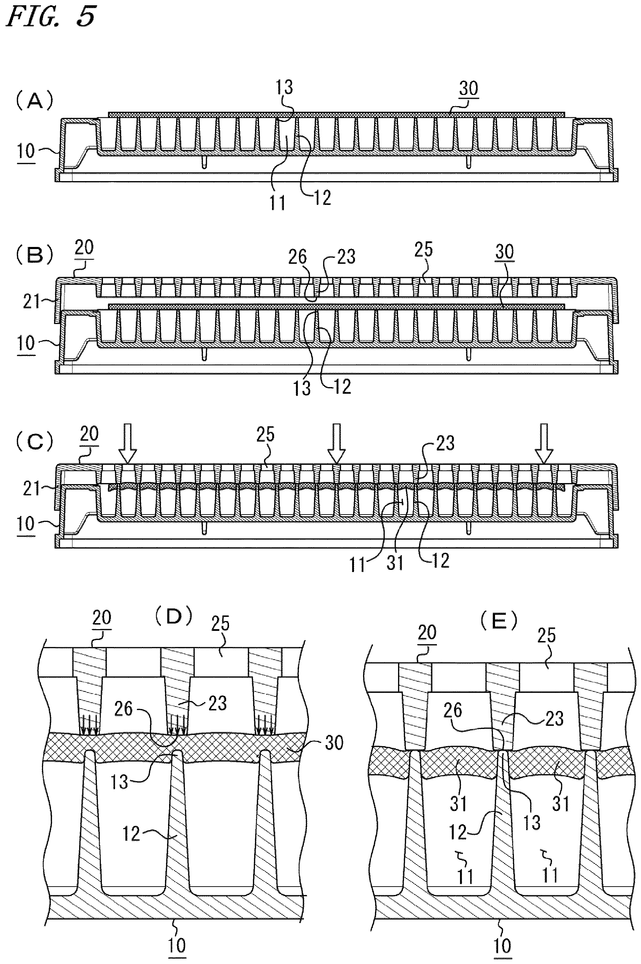

Figs. 5A to 5C are sectional views respectively showing process steps of fragmenting and dispensing a gel plate using the microtiter plate and the pressing tool, and Figs. 5D and 5E are sectional views enlarging a pertinent portion and respectively showing a state during division of the gel plate and a state after division of the gel plate.

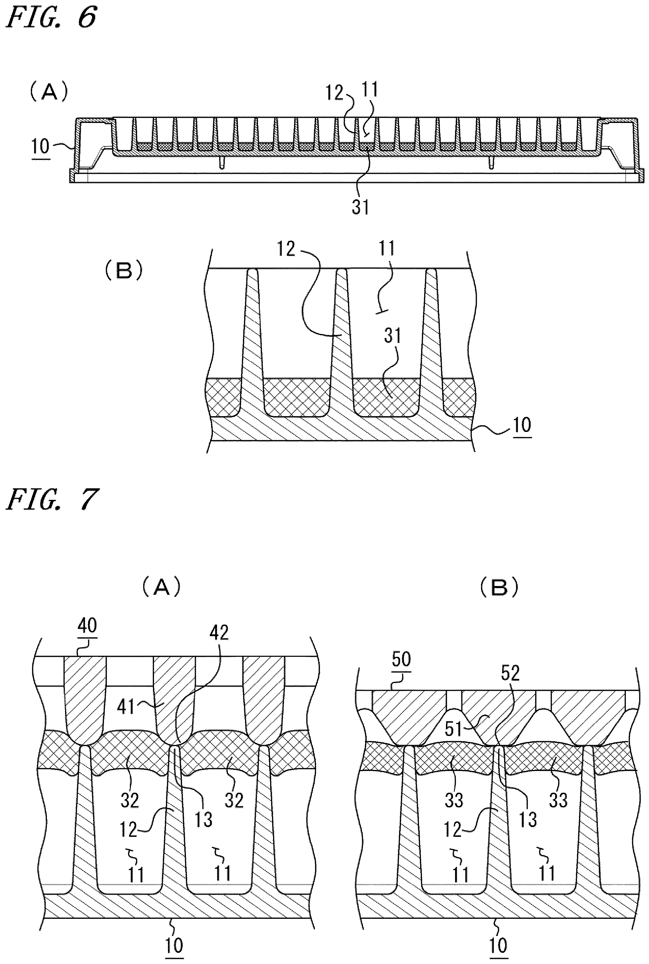

Fig. 6A is a sectional view of the microtiter plate, and Fig. 6B is a sectional view enlarging a pertinent portion of the microtiter plate both showing a state where the gel plate is fragmented and dispensed into concave portions of the microtiter plate by using the microtiter plate and the pressing tool, and then the dispensed gel is deposited on bottom sides of the concave portions by a centrifuge.
Figs. 7A and 7B are each a sectional view enlarging a pertinent portion showing a state after the gel plate has been divided by a pressing tool according to a variation example.
Fig. 8 is a perspective view showing a device according to a prior art (disclosed in Patent Document 5) constituted from a flat plate and a bottomless microtiter plate and a gel plate placed on the flat plate.
Fig. 9 is a sectional view showing a state where a solvent is applied to a gel dispensed into small segments, thereby extracting and analyzing molecules, in the device according to the prior art (disclosed in Patent Document 5) .

### Description of Embodiments

An embodiment of "a fragmentation and dispensing apparatus of a gel plate and a fragmentation and dispensing method of the gel plate" of the present invention will be described below in detail, with reference to Figs. 1 to 7.

First, Fig. 1 shows a microtiter plate 10 to be used in this embodiment.

This microtiter plate 10 is similar to a common microtiter plate with 384 wells (holes) that has hitherto been sold, in appearance. The microtiter plate 10 is a molded product formed of a resin such as polystyrene, and is a plate where a lot of bottomed wells 11 of a square, planar shape are arranged in the form of a matrix.

Then, each well 11 is surrounded by a lattice-shaped partition wall 12. Each well 11 has a U-shaped bottom. The microtiter plate 10 in this embodiment is characterized in that end portions 13 of the lattice-shaped partition wall 12 are formed to be sharp and that an opening portion of each well 11 is formed by sharp ridges.

Next, Fig. 2 shows a pressing tool 20 to be used in this embodiment. The pressing tool 20 is constituted from a frame portion 21 configured to be fit onto a side peripheral surface of the microtiter plate 10 and a pressing surface portion 22 opposed to the upper surface of the microtiter plate 10.

A lattice-shaped partition wall 23 is formed on an inside surface of the pressing surface portion 22 in a state of projecting from the inside surface of the pressing surface portion 22, and an opening 25 is formed in each square region surrounded by the partition wall 23 on an upper surface plate 24 from which the partition wall 23 is erected inward.

Then, the lattice-shaped partition wall 23 of the pressing surface portion 22 is formed in a region opposed to the lattice-shaped partition wall 12 on the side of the microtiter plate 10 when the pressing tool 20 is attached to the microtiter plate 10 by fitting the frame portion 21 onto the side peripheral surface of the microtiter plate 10. Though the end portions of the lattice-shaped partition wall 12 on the side of the microtiter plate 10 are formed to be sharp, end surfaces of the lattice-shaped partition wall 23 of this pressing tool 20 is formed as band-like surfaces each having a predetermined width.

Accordingly, when the pressing tool 20 is attached to the microtiter plate 10, end surfaces 26 of the lattice-shaped partition wall 23 on the side of the pressing tool 20 abut against the sharp end portions 13 of the lattice-shaped partition wall 12 on the side of the microtiter plate 10, as shown in Fig. 3.

Fig. 4 is an enlarged view of a pertinent portion clearly showing the abutment parts. As shown in Fig. 4, reference character "Wc" denotes a width of a section of the sharp end portion 13 of the lattice-shaped partition wall 12 on the side of the microtiter plate 10 corresponding to a cutting edge, and reference character "Wp" denotes a width of the end surface 26 of the lattice-shaped partition wall 23 on the side of the pressing tool 20. A relationship of Wp > Wc is established between the size of the width Wc and the size of the width Wp.

Though the upper limit of the size of the width Wp is also limited due to adherence of a gel plate to be cut, details of the upper limit of the size of the width Wp will be described next with reference to Fig. 5.

Fig. 5 shows fragmentation and dispensing process steps of the gel plate using the microtiter plate 10 and the pressing tool 20.

First, the plate of polyacrylamide gel obtained by two-dimensional electrophoresis for analysis of protein is stained. Then, a stained gel plate 30 of the polyacrylamide gel is placed on a region formed by the lattice-shaped partition wall 12 of the microtiter plate 10, as shown in Fig. 5A.

Specifically, the gel plate 30 has a thickness of about 1 mm, and is placed on a surface formed by the end portions of the lattice-shaped partition wall 12 in a laid-out state.

Next, when the frame portion 21 of the pressing tool 20 is fit onto the side peripheral surface of the microtiter plate 10 and then the pressing tool 20 is pressed down toward the microtiter plate 10 in that state as shown in Fig. 5B, the end surfaces 26 of the lattice-shaped partition wall 23 on the side of the pressing tool 20 move to be opposed to and abut against the end portions 13 of the lattice-shaped partition wall 12 on the side of the microtiter plate 10. During the process of pressing down the pressing tool 20, the end surfaces 26 of the partition wall 23 press the gel plate 30 against the sharp end portions 13 of the partition wall 12, as shown in Fig. 5D.

Accordingly, the gel plate 30 is divided by the sharp ridges of the opening portions of the respective wells 11 arranged in the form of the matrix.

Then, when the gel plate 30 is completely cut in the abutting state between the end surfaces 26 of the lattice-shaped partition wall 23 and the end portions 13 of the lattice-shaped partition wall 12, gel fragments 31 resulting from the division of the gel plate 30 are pressed into the opening portions of the respective wells 11 of the microtiter plate 10, as shown in Figs. 5C and 5E.

That is, a peripheral edge portion of each gel fragment 31 is pressed into the well 11 of the microtiter plate 10 using a band-like surface of the end surface 26 of the lattice-shaped wall 23 of the pressing tool 20 having a width corresponding to the width of (Wp - Wc) /2 (with regard to the widths Wp and Wc, refer to Fig. 4), so that the gel fragment 31 is dispensed into each well 11. Each gel fragment 31 tends to adhere to and remain in an inner wall surface in the vicinity of the opening portion of the well 11.

After the gel fragment 31 has been dispensed into each well 11 of the microtiter plate 10 in this manner, the pressing tool 20 is released from the state of being fitted on the microtiter plate 10. Then, the upper surface of the microtiter plate 10 is released, and a transition is made to analysis of the gel fragments 31 in the respective wells 11.

If an adhesive force between the band-like surface of the pressing tool 20 and the peripheral edge portion of the gel fragment 31 is large during the process of releasing the pressing tool 20, the gel fragment 31 once dispensed into the well 11 is extracted in the state of being adhered to the pressing tool 20, against an adhesive force of the gel fragment 31 to the inner wall surface of the well 11.

For that reason, there is the upper limit to the size of the width Wp of each end surface 26 of the lattice-shaped partition wall 23 on the side of the pressing tool 20. The width Wp of the end surface 26 is set within a range that does not cause the above-mentioned malfunction.

The gel fragment 31 obtained by dividing the gel plate 30 after the stain process is dispensed in each well 11 of the microtiter plate 10 while maintaining a planar positional relationship. The gel fragment 31 colored by the staining is targeted for analysis by in-gel digestion.

Specifically, peptides are collected, and are placed on a target plate, for measurement by a mass spectrometer. The peptides are obtained by fragmentation treatment of the protein by a protease such as trypsin.

In the case of this embodiment, each well 11 is used without alteration as a Petri dish to perform the process of collecting the peptides. However, when the dispensed gel fragment 31 remains in the opening portion of each well 11 as shown in Figs. 5C and 5E, injection of a solution of the protease is obstructed.

The gel fragments 31 may be pressed into the respective wells 11 by a pipette or a thin stick in advance in order to address this problem. However, the pressing operation for a lot of the wells 11 is laborious. Thus, the microtiter plate 10 is set in a centrifuge when the gel fragment 31 has been dispensed into each well 11 of the microtiter plate 10 as shown in Figs. 5C and 5E. Then, by causing a centrifugal force to act on the gel fragment 31 toward a bottom portion of each well 11, the gel fragment 31 may be deposited on the side of the bottom portion of each well 11, as shown in Figs. 6A and 6B.

It is arbitrary whether this process is to be performed in a state where the pressing tool 20 is attached to the microtiter plate 10.

In the above-mentioned embodiment, each end surface 26 of the lattice-shaped partition wall 23 on the side of the pressing tool 20 is formed as the planar surface as shown in Fig. 4. However, the end surface 26 is not necessarily limited to the planar surface, and the end surface 26 may be a curved surface such as an arcuate surface.

Fig. 7A shows a state where the gel plate 30 is divided by using a pressing tool 40 according to a variation example of the embodiment. The microtiter plate 10, however, has a similar configuration as that in the above-mentioned embodiment.

By locally pressing the gel plate 30 against the sharp end portions 13 of the lattice-shaped partition wall 12 on the side of the microtiter plate 10 by articulate surfaces 42 formed at the end portions of a lattice-shaped wall portion 41 of the pressing tool 40, the gel plate 30 is sandwiched between the microtiter plate 10 and the pressing tool 40 and is then cut. Peripheral edge portions of gel fragments 32 after the division are respectively pressed into the wells 11 of the microtiter plate 10, thereby dispensing the gel fragments 32.

Meanwhile, in this variation example, the articulate surfaces 42 of the pressing tool 40 locally press the gel plate 30. When the curvature radius of each articulate surface 42 is too small, the articulate surface 42 serves as a cutting edge to cut into the gel plate 30. Conversely, when the curvature radius of the articulate surface 42 is too large, a contact area between the articulate surface 42 and the gel plate 30 increases. In either case, the gel fragment 32 is adhered to the articulate surface 42 when the pressing tool 40 is released from the microtiter plate 10, thereby causing obstruction of dispensing of the gel fragment 32 into the well 11.

Accordingly, the curvature radius of each articulate surface 42 of the pressing tool 40 is set within a range that does not cause the malfunction, also in view of adherence of the gel plate 30.

In the above-mentioned embodiment or the above-mentioned variation example, a pressing region for the gel plate on the side of the pressing tool 20 is configured as the lattice-shaped partition wall 23 or the lattice-shaped wall portion 41. The pressing region for the gel plate does not necessarily need to be configured in the form of a wall. The pressing region for the gel plate may be a lattice-shaped convex portion 51 of the pressing tool 50 that has risen to a surface opposed to the microtiter plate 10, as shown in Fig. 7B.

Tip sections 52 of the convex portion 51 in this case abut against the end portions 13 of the lattice-shaped partition wall 12 on the side of the microtiter plate 10. Each tip section 52 may have a flat surface or a curved surface. In either case, the tip section 52 must have a condition for avoiding adhesion between the gel fragments 33 and the pressing tool 50 shown in the above-mentioned embodiment or the above-mentioned variation example.

### Industrial Applicability

The present invention may be applied to an apparatus and a method for transferring, to the microtiter plate, the gel plate after electrophoresis that is a method of analyzing protein and DNA, and then treating the protein and the DNA.

### Reference Signs List

10 microtiter plate
11 well
12 partition wall
13 end portion of partition wall
20 pressing tool
21 frame portion
22 pressing surface portion
23 partition wall
24 upper surface plate
25 opening
26 end surface of partition wall
30 gel plate
31 gel fragment
40 pressing tool
41 wall portion
42 articulate surface
50 pressing tool
51 convex portion
52 tip portion
101 flat plate
102 gel plate
103 bottomless microtiter plate (frame)
103a thin wall
104 gel fragment
105 solvent

## Claims

1. A fragmentation and dispensing apparatus of a gel plate, wherein the fragmentation and dispensing apparatus comprises:
a microtiter plate (10) including a lattice-shaped partition wall (12) with concave portions (11) thereof surrounded by the lattice-shaped partition wall (12) and arranged in the form of a matrix, end portions (13) of the lattice-shaped partition wall (12) being formed to be sharp; and
a pressing tool (20) including fitting means with respect to the microtiter plate (10) and including a lattice-shaped wall portion (41) or a lattice-shaped convex portion (51) formed thereon, the lattice-shaped wall portion (41) or the lattice-shaped convex portion (51) having a flat surface or a curved surface configured to be opposed to and abut against each end portion of the lattice-shaped partition wall (12) of the microtiter plate (10) when the pressing tool (20) is fitted onto the microtiter plate (10); and
by fitting the pressing tool (20) onto the microtiter plate (10) and pressing a gel plate (30) against the microtiter plate (10) with the gel plate (30) placed on the lattice-shaped partition wall (12) of the microtiter plate (10), the gel plate (30) is sandwiched and cut between the sharp end portions of the partition wall on the side of the microtiter plate and the surfaces (flat surfaces or curved surfaces) of the wall portion or the convex portion on the side of the pressing tool that have abutted against the sharp end portions and is thereby divided into fragments (31) and the fragments are then dispensed into the respective concave portions (11) of the microtiter plate (10).

2. The fragmentation and dispensing apparatus of a gel plate according to claim 1, wherein
an abutment part of the lattice-shaped wall portion or the lattice-shaped convex portion of the pressing tool configured to abut against each end portion of the lattice-shaped partition wall of the microtiter plate is formed as a flat end surface having a predetermined width, and the width of the end surface is larger than a width of a section of the end portion of the lattice-shaped partition wall of the microtiter plate corresponding to a cutting edge, and the width of the end surface is set within a range that does not cause adhesion of a gel to the pressing tool due to adherence of the gel constituting the gel plate and divided into the fragments when the pressing tool is released from the state of being fitted on the microtiter plate.

3. The fragmentation and dispensing apparatus of a gel plate according to claim 1, wherein an abutment part of the lattice-shaped wall portion or the lattice-shaped convex portion of the pressing tool configured to abut against each end portion of the lattice-shaped partition wall of the microtiter plate is formed as a convex articulate surface, and a curvature of the articulate surface is set within a range that does not cause adhesion of a gel to the pressing tool due to adherence of the gel constituting the gel plate and divided into the fragments when the pressing tool is released from the state of being fitted on the microtiter plate.

4. The fragmentation and dispensing apparatus of a gel plate according to claim 1, 2, or 3, wherein
the fitting means of the pressing tool with respect to the microtiter plate is a mechanism whereby an outer peripheral frame portion (21) of the pressing tool is fit onto an outer peripheral side surface of the microtiter plate.

5. A fragmentation and dispensing method of a gel plate using a microtiter plate (10) and a pressing tool (20), the microtiter plate (10) including a lattice-shaped partition wall (12) with concave portions (11) thereof surrounded by the lattice-shaped partition wall (12) and arranged in the form of a matrix, end portions (13) of the lattice-shaped partition wall (12) being formed to be sharp, the pressing tool (20) including fitting means with respect to the microtiter plate (10) and including a lattice-shaped wall portion (41) or a lattice-shaped convex portion (51) formed thereon, the lattice-shaped wall portion (41) or the lattice-shaped convex portion (51) having a flat surface or a curved surface configured to be opposed to and abut against each end portion (13) of the lattice-shaped partition wall (12) of the microtiter plate (10) when the pressing tool (20) is fitted onto the microtiter plate (10), the fragmentation and dispensing method comprising:
a first step of placing a gel plate (30) on the end portions (13) of the lattice-shaped partition wall (12) of the microtiter plate (10); and
a second step of fitting the pressing tool (20) onto the microtiter plate (10) to press the gel plate (30) against the microtiter plate (10), such that the gel plate is sandwiched and cut between the sharp end portions of the partition wall on the side of the microtiter plate and the surfaces (flat surfaces or curved surfaces) of the wall portion or the convex portion on the side of the pressing tool that have abutted against the sharp end portions, thereby dividing the gel plate (30) placed in the first step into fragments (31) and then dispensing the fragments (31) into the respective concave portions (11) of the microtiter plate (10).

6. The fragmentation and dispensing method of a gel plate according to claim 5, wherein
the fitting means of the pressing tool with respect to the microtiter plate is a mechanism whereby an outer peripheral frame portion (21) of the pressing tool is fit onto an outer peripheral side surface of the microtiter plate.

7. The fragmentation and dispensing method of a gel plate according to claim 5, wherein
an abutment part of the lattice-shaped wall portion or the lattice-shaped convex portion of the pressing tool configured to abut against each end portion of the lattice-shaped partition wall of the microtiter plate is formed as a flat end surface having a predetermined width, and the width of the end surface is larger than a width of a section of the end portion of the lattice-shaped partition wall of the microtiter plate corresponding to a cutting edge, and the width of the end surface is set within a range that does not cause adhesion of a gel to the pressing tool due to adherence of the gel constituting the gel plate and divided into the fragments when the pressing tool is released from the state of being fitted on the microtiter plate.

8. The fragmentation and dispensing method of a gel plate according to claim 7, wherein
the fitting means of the pressing tool with respect to the microtiter plate is a mechanism whereby an outer peripheral frame portion (21) of the pressing tool is fit onto an outer peripheral side surface of the microtiter plate.

9. The fragmentation and dispensing method of a gel plate according to claim 5, wherein
an abutment part of the lattice-shaped wall portion or the lattice-shaped convex portion of the pressing tool configured to abut against each end portion of the lattice-shaped partition wall of the microtiter plate is formed as a convex articulate surface, and a curvature of the articulate surface is set within a range that does not cause adhesion of a gel to the pressing tool due to adherence of the gel constituting the gel plate and divided into the fragments when the pressing tool is released from the state of being fitted on the microtiter plate.

10. The fragmentation and dispensing method of a gel plate according to claim 9, wherein
the fitting means of the pressing tool with respect to the microtiter plate is a mechanism whereby an outer peripheral frame portion (21) of the pressing tool is fit onto an outer peripheral side surface of the microtiter plate.

11. The fragmentation and dispensing method of a gel plate according to claim 5, 6, 7, 8, 9, or 10, wherein
by causing a centrifugal force to act on the fragments of the gel plate dispensed into the respective concave portions of the microtiter plate in a direction toward bottom portions of the respective concave portions, the fragments of the gel plate are deposited on bottom sides of the respective concave portions.

## Patentansprüche

1. Fragmentierungs- und Ausgabeapparatur bzw. -Dispenserapparatur für eine Gelplatte, wobei die Fragmentierungs- und Ausgabeapparatur umfasst:
eine Mikrotiterplatte (10), welche eine gitterförmige Trennwand (12) mit konkaven Abschnitten (11) davon einschließt, welche von der gitterförmigen Trennwand (12) umgeben sind und in Form einer Matrix angeordnet sind, wobei Endabschnitte (13) der gitterförmigen Trennwand (12) als scharf ausgebildet sind; und
ein Presswerkzeug (20), welches Fitting-Einrichtungen bezüglich der Mikrotiterplatte (10) einschließt und einen gitterförmigen Wandabschnitt (41) oder einen gitterförmigen konvexen Abschnitt (51), welcher darauf ausgebildet ist, einschließt, wobei der gitterförmige Wandabschnitt (41) oder der gitterförmige konvexe Abschnitt (51) mit einer flachen Oberfläche oder einer gekrümmten Oberfläche konfiguriert ist, um jedem Endabschnitt der gitterförmigen Trennwand (12) der Mikrotiterplatte (10) gegenüber zu liegen und an diesen anzugrenzen, wenn das Presswerkzeug (20) auf die Mikrotiterplatte (10) angebracht bzw. eingepasst wird; und
durch Anbringen bzw. Einpassen des Presswerkzeugs (20) auf die Mikrotiterplatte (10) und Pressen einer Gelplatte (30) gegen die Mikrotiterplatte (10), wobei die Gelplatte (30) auf der gitterförmigen Trennwand (12) der Mikrotiterplatte (10) platziert ist, die Gelplatte (30) zwischen die scharfen Endabschnitte der Trennwand auf der Seite der Mikrotiterplatte und den Oberflächen (flachen Oberflächen oder gekrümmten Oberflächen) des Wandabschnitts oder des konvexen Abschnitts auf der Seite des Presswerkzeugs, welche an die scharfen Endabschnitte angrenzen, eingezwängt und geschnitten wird und dadurch in Fragmente (31) geteilt wird und die Fragmente dann in die jeweiligen konkaven Abschnitte (11) der Mikrotiterplatte (10) ausgegeben werden.

2. Fragmentierungs- und Ausgabeapparatur für eine Gelplatte gemäß Anspruch 1, wobei ein Angrenzungsteil des gitterförmigen Wandabschnitts oder des gitterförmigen konvexen Abschnitts des Presswerkzeugs, welches konfiguriert ist, um gegen jeden Endabschnitt der gitterförmigen Trennwand der Mikrotiterplatte anzugrenzen, als eine flache Endoberfläche mit einer vorbestimmten Breite ausgebildet ist, und die Breite der Endoberfläche größer als eine Breite eines Bereichs des Endabschnitts der gitterförmigen Trennwand der Mikrotiterplatte entsprechend einer Schnittkante ist, und die Breite der Endoberfläche innerhalb eines Bereichs gesetzt wird, der nicht eine Adhäsion eines Gels an das Presswerkzeug aufgrund eines Anhaftens des Gels, welches die Gelplatte darstellt und in die Fragmente geteilt wird, wenn das Presswerkzeug aus dem Zustand des Angebracht-Seins auf der Mikrotiterplatte freigegeben wird, verursacht.

3. Fragmentierungs- und Ausgabeapparatur für eine Gelplatte gemäß Anspruch 1, wobei ein Angrenzungsteil des gitterförmigen Wandabschnitts oder des gitterförmigen konvexen Abschnitts des Presswerkzeugs, welches konfiguriert ist, um gegen jeden Endabschnitt der gitterförmigen Trennwand der Mikrotiterplatte anzugrenzen, als eine konvexe artikulierte Oberfläche ausgebildet ist, und eine Krümmung der artikulierten Oberfläche innerhalb eines Bereichs gesetzt wird, der nicht eine Adhäsion eines Gels an das Presswerkzeug aufgrund eines Anhaftens des Gels, welches die Gelplatte darstellt und in die Fragmente geteilt wird, wenn das Presswerkzeug aus dem Zustand des Angebracht-Seins auf der Mikrotiterplatte freigegeben wird, verursacht.

4. Fragmentierungs- und Ausgabeapparatur für eine Gelplatte gemäß Anspruch 1, 2 oder 3, wobei es sich bei den Fitting-Einrichtungen des Presswerkzeugs bezüglich der Mikrotiterplatte um einen Mechanismus handelt, bei dem ein äußerer peripherer Rahmenabschnitt (21) des Presswerkzeugs auf eine äußere periphere Seitenoberfläche der Mikrotiterplatte angebracht bzw. eingepasst wird.

5. Fragmentierungs- und Ausgabemethode bzw. -Dispensermethode für eine Gelplatte unter Verwendung einer Mikrotiterplatte (10) und eines Presswerkzeugs (20), wobei die Mikrotiterplatte (10) eine gitterförmige Trennwand (12) mit konkaven Abschnitten (11) davon einschließt, welche von der gitterförmigen Trennwand (12) umgeben sind und in Form einer Matrix angeordnet sind, wobei Endabschnitte (13) der gitterförmigen Trennwand (12) als scharf ausgebildet sind, wobei das Presswerkzeug (20) Fitting-Einrichtungen bezüglich der Mikrotiterplatte (10) einschließt und einen gitterförmigen Wandabschnitt (41) oder einen gitterförmigen konvexen Abschnitt (51), welcher darauf ausgebildet ist, einschließt, wobei der gitterförmige Wandabschnitt (41) oder der gitterförmige konvexe Abschnitt (51) mit einer flachen Oberfläche oder einer gekrümmten Oberfläche konfiguriert ist, um jedem Endabschnitt (13) der gitterförmigen Trennwand (12) der Mikrotiterplatte (10) gegenüber zu liegen und an diesen anzugrenzen, wenn das Presswerkzeug (20) auf die Mikrotiterplatte (10) angebracht bzw. eingepasst wird, wobei die Fragmentierungs- und Ausgabemethode umfasst:
einen ersten Schritt des Platzierens einer Gelplatte (30) auf die Endabschnitte (13) der gitterförmigen Trennwand (12) der Mikrotiterplatte (10); und
einen zweiten Schritt des Anbringens bzw. Einpassens des Presswerkzeugs (20) auf die Mikrotiterplatte (10), um die Gelplatte (30) gegen die Mikrotiterplatte (10) zu pressen, derart, dass die Gelplatte zwischen den scharfen Endabschnitten der Trennwand auf der Seite der Mikrotiterplatte und den Oberflächen (flachen Oberflächen oder gekrümmten Oberflächen) des Wandabschnitts oder des konvexen Abschnitts auf der Seite des Presswerkzeugs, welche an die scharfen Endabschnitte angrenzen, eingezwängt und geschnitten wird, wodurch die im ersten Schritt platzierte Gelplatte (30) in Fragmente (31) geteilt wird und dann die Fragmente (31) in die jeweiligen konkaven Abschnitte (11) der Mikrotiterplatte (10) ausgegeben werden.

6. Fragmentierungs- und Ausgabemethode für eine Gelplatte gemäß Anspruch 5, wobei es sich bei den Fitting-Einrichtungen des Presswerkzeugs bezüglich der Mikrotiterplatte um einen Mechanismus handelt, bei dem ein äußerer peripherer Rahmenabschnitt (21) des Presswerkzeugs auf eine äußere periphere Seitenoberfläche der Mikrotiterplatte angebracht bzw. eingepasst wird.

7. Fragmentierungs- und Ausgabemethode für eine Gelplatte gemäß Anspruch 5, wobei ein Angrenzungsteil des gitterförmigen Wandabschnitts oder des gitterförmigen konvexen Abschnitts des Presswerkzeugs, welches konfiguriert ist, um gegen jeden Endabschnitt der gitterförmigen Trennwand der Mikrotiterplatte anzugrenzen, als eine flache Endoberfläche mit einer vorbestimmten Breite ausgebildet ist, und die Breite der Endoberfläche größer als eine Breite eines Bereichs des Endabschnitts der gitterförmigen Trennwand der Mikrotiterplatte entsprechend einer Schnittkante ist, und die Breite der Endoberfläche innerhalb eines Bereichs gesetzt wird, der nicht eine Adhäsion eines Gels an das Presswerkzeug aufgrund eines Anhaftens des Gels, welches die Gelplatte darstellt und in die Fragmente geteilt wird, wenn das Presswerkzeug aus dem Zustand des Angebracht-Seins auf der Mikrotiterplatte freigegeben wird, verursacht.

8. Fragmentierungs- und Ausgabemethode für eine Gelplatte gemäß Anspruch 7, wobei es sich bei den Fitting-Einrichtungen des Presswerkzeugs bezüglich der Mikrotiterplatte um einen Mechanismus handelt, bei dem ein äußerer peripherer Rahmenabschnitt (21) des Presswerkzeugs auf eine äußere periphere Seitenoberfläche der Mikrotiterplatte angebracht bzw. eingepasst wird.

9. Fragmentierungs- und Ausgabemethode für eine Gelplatte gemäß Anspruch 5, wobei ein Angrenzungsteil des gitterförmigen Wandabschnitts oder des gitterförmigen konvexen Abschnitts des Presswerkzeugs, welches konfiguriert ist, um gegen jeden Endabschnitt der gitterförmigen Trennwand der Mikrotiterplatte anzugrenzen, als eine konvexe artikulierte Oberfläche ausgebildet ist, und eine Krümmung der artikulierten Oberfläche innerhalb eines Bereichs gesetzt wird, der nicht eine Adhäsion eines Gels an das Presswerkzeug aufgrund eines Anhaftens des Gels, welches die Gelplatte darstellt und in die Fragmente geteilt wird, wenn das Presswerkzeug aus dem Zustand des Angebracht-Seins auf der Mikrotiterplatte freigegeben wird, verursacht.

10. Fragmentierungs- und Ausgabemethode für eine Gelplatte gemäß Anspruch 9, wobei es sich bei den Fitting-Einrichtungen des Presswerkzeugs bezüglich der Mikrotiterplatte um einen Mechanismus handelt, bei dem ein äußerer peripherer Rahmenabschnitt (21) des Presswerkzeugs auf eine äußere periphere Seitenoberfläche der Mikrotiterplatte angebracht bzw. eingepasst wird.

11. Fragmentierungs- und Ausgabemethode für eine Gelplatte gemäß Anspruch 5, 6, 7, 8, 9 oder 10, wobei durch Veranlassen bzw. Herbeiführen einer Zentrifugalkraft, die auf die in die jeweiligen konkaven Abschnitte der Mikrotiterplatte ausgegebenen Fragmente der Gelplatte wirkt, in eine Richtung zu Unterabschnitten der jeweiligen konkaven Abschnitte die Fragmente der Gelplatte auf Unterseiten der jeweiligen konkaven Abschnitte abgeschieden werden.

## Revendications

1. Un appareil de fragmentation et de distribution d'une plaque de gel, l'appareil de fragmentation et de distribution comprenant :
une plaque de microtitration (10) comprenant une paroi de séparation en forme de treillis (12) avec des parties concaves (11) de celle-ci entourées par la paroi de séparation en forme de treillis (12) et disposées en forme de matrice, des parties extrêmes (13) de la paroi de séparation en forme de treillis (12) étant conçues pour être tranchantes ; et
un outil de pressage (20) comprenant un moyen d'application par rapport à la plaque de microtitration (10) et comprenant une partie de paroi en forme de treillis (41) ou une partie convexe en forme de treillis (51) formée dessus, la partie de paroi en forme de treillis (41) ou la partie convexe en forme de treillis (51) ayant une surface plate ou une surface courbe configurée pour être opposée à et venir en butée contre chaque partie extrême de la paroi de séparation en forme de treillis (12) de la plaque de microtitration (10) lorsque l'outil de pressage (20) est appliqué sur la plaque de microtitration (10) ; et
en appliquant l'outil de pressage (20) sur la plaque de microtitration (10) et en pressant une plaque de gel (30) contre la plaque de microtitration (10) avec la plaque de gel (30) posée sur la paroi de séparation en forme de treillis (12) de la plaque de microtitration (10), la plaque de gel (30) étant prise en sandwich et coupée entre les parties extrêmes tranchantes de la paroi de séparation du côté de la plaque de microtitration et les surfaces (surfaces plates ou surfaces courbes) de la partie de paroi ou de la partie convexe du côté de l'outil de pressage qui est venu en butée contre les parties extrêmes tranchantes et est ainsi divisée en fragments (31) et les fragments sont ensuite distribués dans les parties concaves respectives (11) de la plaque de microtitration (10).

2. L'appareil de fragmentation et de distribution d'une plaque de gel selon la revendication 1, dans lequel
une portion de butée de la partie de paroi en forme de treillis ou de la partie convexe en forme de treillis de l'outil de pressage conçue pour venir en butée contre chaque partie extrême de la paroi de séparation en forme de treillis de la plaque de microtitration est conformée en surface extrême plate ayant une largeur prédéterminée, et la largeur de la surface extrême est supérieure à une largeur d'une section de la partie extrême de la paroi de séparation en forme de treillis de la plaque de microtitration correspondant à un bord tranchant, et la largeur de la surface extrême est située dans une plage qui ne provoque pas l'adhésion d'un gel à l'outil de pressage, due à l'adhérence du gel qui constitue la plaque de gel et est divisé en fragments, lorsque l'outil de pressage quitte l'état d'application sur la plaque de microtitration.

3. L'appareil de fragmentation et de distribution d'une plaque de gel selon la revendication 1, dans lequel une portion de butée de la partie de paroi en forme de treillis ou de la partie convexe en forme de treillis de l'outil de pressage conçue pour venir en butée contre chaque partie extrême de la paroi de séparation en forme de treillis de la plaque de microtitration est conformée en surface articulée convexe, et une courbure de la surface articulée est située dans une plage qui ne provoque pas l'adhésion d'un gel à l'outil de pressage, due à l'adhérence du gel qui constitue la plaque de gel et est divisé en fragments, lorsque l'outil de pressage quitte l'état d'application sur la plaque de microtitration.

4. L'appareil de fragmentation et de distribution d'une plaque de gel selon la revendication 1, 2 ou 3, dans lequel
le moyen d'application de l'outil de pressage par rapport à la plaque de microtitration est un mécanisme par l'intermédiaire duquel une partie de cadre périphérique extérieur (21) de l'outil de pressage est appliquée sur une surface latérale périphérique extérieure de la plaque de microtitration.

5. Un procédé de fragmentation et de distribution d'une plaque de gel utilisant une plaque de microtitration (10) et un outil de pressage (20), la plaque de microtitration (10) comprenant une paroi de séparation en forme de treillis (12) avec des parties concaves (11) de celle-ci entourées par la paroi de séparation en forme de treillis (12) et disposées en forme de matrice, des parties extrêmes (13) de la paroi de séparation en forme de treillis (12) étant conçues pour être tranchantes, l'outil de pressage (20) comprenant un moyen d'application par rapport à la plaque de microtitration (10) et comprenant une partie de paroi en forme de treillis (41) ou une partie convexe en forme de treillis (51) formée dessus, la partie de paroi en forme de treillis (41) ou la partie convexe en forme de treillis (51) ayant une surface plate ou une surface courbe configurée pour être opposée à et venir en butée contre chaque partie extrême (13) de la paroi de séparation en forme de treillis (12) de la plaque de microtitration (10) lorsque l'outil de pressage (20) est appliqué sur la plaque de microtitration (10), le procédé de fragmentation et de distribution comprenant :
une première étape consistant à poser une plaque de gel (30) sur les parties extrêmes (13) de la paroi de séparation en forme de treillis (12) de la plaque de microtitration (10) ; et
une deuxième étape consistant à appliquer l'outil de pressage (20) sur la plaque de microtitration (10) pour presser la plaque de gel (30) contre la plaque de microtitration (10), de façon que la plaque de gel soit prise en sandwich et coupée entre les parties extrêmes tranchantes de la paroi de séparation du côté de la plaque de microtitration et les surfaces (surfaces plates ou surfaces courbes) de la partie de paroi ou de la partie convexe du côté de l'outil de pressage qui est venu en butée contre les parties extrêmes tranchantes,
divisant ainsi la plaque de gel (30), posée dans la première étape, en fragments (31) et distribuant ensuite les fragments (31) dans les parties concaves respectives (11) de la plaque de microtitration (10).

6. Le procédé de fragmentation et de distribution d'une plaque de gel selon la revendication 5, dans lequel
le moyen d'application de l'outil de pressage par rapport à la plaque de microtitration est un mécanisme par l'intermédiaire duquel une partie de cadre périphérique extérieur (21) de l'outil de pressage est appliquée sur une surface latérale périphérique extérieure de la plaque de microtitration.

7. Le procédé de fragmentation et de distribution d'une plaque de gel selon la revendication 5, dans lequel
une portion de butée de la partie de paroi en forme de treillis ou de la partie convexe en forme de treillis de l'outil de pressage conçue pour venir en butée contre chaque partie extrême de la paroi de séparation en forme de treillis de la plaque de microtitration est conformée en surface extrême plate ayant une largeur prédéterminée, et la largeur de la surface extrême est supérieure à une largeur d'une section de la partie extrême de la paroi de séparation en forme de treillis de la plaque de microtitration correspondant à un bord tranchant, et la largeur de la surface extrême est située dans une plage qui ne provoque pas l'adhésion d'un gel à l'outil de pressage, due à l'adhérence du gel qui constitue la plaque de gel et est divisé en fragments, lorsque l'outil de pressage quitte l'état d'application sur la plaque de microtitration.

8. Le procédé de fragmentation et de distribution d'une plaque de gel selon la revendication 7, dans lequel
le moyen d'application de l'outil de pressage par rapport à la plaque de microtitration est un mécanisme par l'intermédiaire duquel une partie de cadre périphérique extérieur (21) de l'outil de pressage est appliquée sur une surface latérale périphérique extérieure de la plaque de microtitration.

9. Le procédé de fragmentation et de distribution d'une plaque de gel selon la revendication 5, dans lequel
une portion de butée de la partie de paroi en forme de treillis ou de la partie convexe en forme de treillis de l'outil de pressage conçue pour venir en butée contre chaque partie extrême de la paroi de séparation en forme de treillis de la plaque de microtitration est conformée en surface articulée convexe, et une courbure de la surface articulée est située dans une plage qui ne provoque pas l'adhésion d'un gel à l'outil de pressage, due à l'adhérence du gel qui constitue la plaque de gel et est divisé en fragments, lorsque l'outil de pressage quitte l'état d'application sur la plaque de microtitration.

10. Le procédé de fragmentation et de distribution d'une plaque de gel selon la revendication 9, dans lequel
le moyen d'application de l'outil de pressage par rapport à la plaque de microtitration est un mécanisme par l'intermédiaire duquel une partie de cadre périphérique extérieur (21) de l'outil de pressage est appliquée sur une surface latérale périphérique extérieure de la plaque de microtitration.

11. Le procédé de fragmentation et de distribution d'une plaque de gel selon la revendication 5, 6, 7, 8, 9 ou 10, dans lequel
en exerçant une force centrifuge sur les fragments de la plaque de gel distribués dans les parties concaves respectives de la plaque de microtitration en direction de parties de fond des parties concaves respectives, les fragments de la plaque de gel sont déposés sur des côtés de fond des parties concaves respectives.
